# NEUE EUROPÄISCHE PATENTSCHRIFT

(11) **EP 0 872 729 B2**
(45) Veröffentlichungstag und Bekanntmachung des Hinweises auf die Entscheidung über den Einspruch: **29.08.2012**
(45) Hinweis auf die Patenterteilung: 27.07.2005
(21) Anmeldenummer: 98890110.4
(22) Anmeldetag: 16.04.1998
(51) Int. Cl.: G01N 27/327, C12Q 1/00

(54) **Biosensor mit permeabler Deckmembran aus Polyvinylchlorid-Copolymer**
Biosensor with a permeable polyvinylchloride outer membrane
Biocapteur avec une membrane copolymère de chlorure de vinyle perméable

(30) Priorität: 17.04.1997 AT 66497
(43) Veröffentlichungstag der Anmeldung: 21.10.1998
(73) Patentinhaber: F. Hoffmann-La Roche AG, 4070 Basel (CH)
(72) Erfinder: Offenbacher, Helmut, 8020 Graz (AT); Pestitschek, Gabriela, 8073 Feldkirchen (AT); Schaffar, Bernhard Peter Harald, 8045 Graz (AT); Dolezal, Andreas Martin, 8020 Graz (AT); Wiedner, Nicole, 8020 Graz (AT)
(74) Vertreter: Schwarz, Albin

(56) Entgegenhaltungen:
- EP-A- 0 707 064
- EP-A1- 0 503 943
- WO-A-92/13271
- GB-A- 1 415 597
- JP-A- 04 370 755
- US-A- 4 214 968
- US-A- 4 900 449
- US-A- 5 540 828
- MEYERHOFF M. E. ET AL: "New polymeric membrane materials for fabricating potentiometric ion- and bioselective sensors." POLYMERIC MATERIALS SCIENCE AND ENGINEERING, Bd. 64, 1991, Seiten 292-293, XP002073355
- GLYN DAVIES, O. ET AL: "Optimisation of poly(vinylchloride) matrix membrane ion-selective electrodes for ammonium ions." THE ANALYST, Bd. 113, Nr. 3, März 1988, Seiten 487-500, XP002073356 london
- BENMAKROHA, Y. ET AL: "Poly(vinylchloride), polysulfone and sulfonated polyether-ether sulfone composite membranes for glucose and hydrogen peroxide perm-selectivity in amperometric biosensors." THE ANALYST, Bd. 121, Nr. 4, April 1996, Seiten 521-526, XP002073357

## Beschreibung

Die vorliegende Erfindung betrifft einen Sensor (Biosensor) zur Bestimmung der Konzentration und zum Nachweis eines Enzymsubstrates in einer flüssigen Probe, welcher Sensor ein Enzym, welches mit dem Enzymsubstrat unter Bildung einer direkt oder indirekt detektierbaren Substanz reagieren kann, eine Detektionsvorrichtung und eine für das Enzymsubstrat permeable Deckmembran bzw. Deckschicht aus einem Polymer aufweist.

Sensoren, ausgebildet als amperometrische Sensoren bzw. Enzymelektroden oder als optische Sensoren (Optoden), werden heutzutage verbreitet eingesetzt, um bestimmte Substanzen, wie z.B. Glucose, Sauerstoff, CO₂, in Blut und anderen Körperflüssigkeiten quantitativ zu bestimmen bzw. nachzuweisen.

Aufbau und Funktion einer amperometrischen Enzymelektrode ist beispielsweise aus der EP-A 0 603 154 der Anmelderin oder aus M. Meyerhoff, 'Polymeric Materials Science and Engineering', Bd. 64, 1991, Seiten 292-293 bekannt. Aufbau und Funktion einer Optode ist beispielsweise aus Biosensors & Bioelectronics 5 (1990) 137-148 bekannt.

Amperometrische Sensoren zur Bestimmung von Glucose, Laktat oder Creatinin werden vorzugsweise mit Oxidoreduktasen und einer Detektionsvorrichtung zur Wasserstoffperoxidbestimmung (Basiselektrode) gebaut. Die Funktionsweise ist dabei so, daß die Oxidase, z.B. Glucoseoxidase, Laktatoxidase und Sarkosinoxidase, den zu bestimmenden Analyten mit Sauerstoff in das korrespondierende Oxidationsprodukt und Wasserstoffperoxid oxidiert, wobei die Konzentration an gebildetem Wasserstoffperoxid der Analytkonzentration proportional ist und durch anodische Oxidation bei ca. 650 mV gegen Ag/AgCl an Edelmetall- oder Carbonelektroden (Basiselektrode) gemessen wird. Alternativ kann auch an katalytisch wirksamen Elektroden, wie platinisiertem Carbon und Mangandioxid, bei reduzierter Oxidationsspannung (ca. 300 mV) gemessen werden.

Weitere Möglichkeiten der Bestimmung sind die Messung des Sauerstoffverbrauchs oder die Verwendung von Mediatoren, welche anstelle des Wasserstoffperoxids an der Elektrode oxidativ gemessen werden können und auch in oxidierter Form als Ersatz für Sauerstoff dienen.

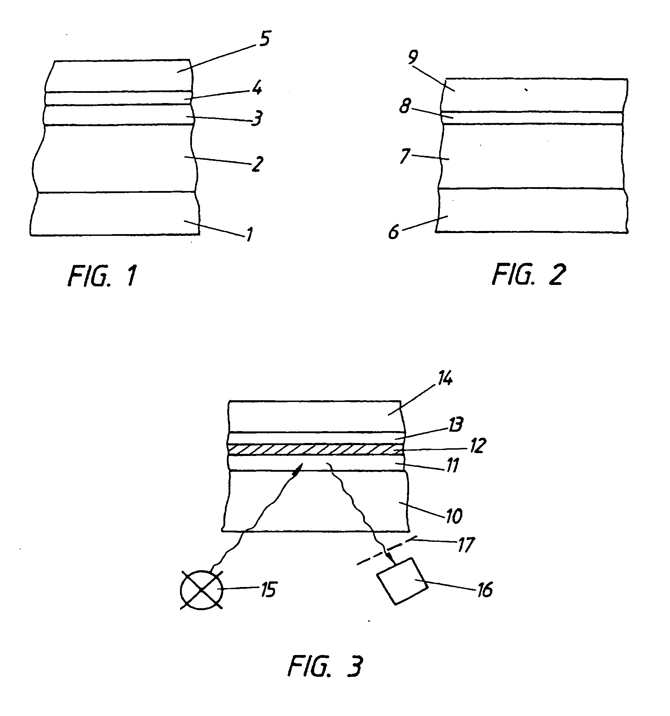

Der klassische amperometrische Sensor besteht aus 4 Schichten: einer Basiselektrode, einer Interferenzsperrschicht, einer Enzymschicht und einer Deckmembran bzw. Deckschicht. Dieser Aufbau ist in der beigefügten Figur 1 schematisch dargestellt, wobei die Bezugsziffer 1 eine elektrisch leitende Bahn, z.B. aus Silber, bezeichnet, die auf einem Träger (nicht dargestellt) aufgebracht ist. Die Basiselektrode 2 ist auf die elektrisch leitende Bahn 1 aufgebracht. Über der Basiselektrode 2 befindet sich die Interferenzsperrschicht 3, die Enzymschicht 4 und die Deckmembran 5. Die Deckmembran 5 steht mit der Probe in Kontakt.

Die Interferenzsperrschicht 3 eines amperometrischen Sensors dient dazu, alle elektroaktiven Substanzen der Probe, z.B. Paracetamol, Harnsäure, Ascorbinsäure, welche direkt an der Elektrodenoberfläche oxidiert werden können und somit falsche, erhöhte Signale geben, von der Elektrode fernzuhalten. Als Interferenzsperrschicht werden gerne Schichten aus Celluloseacetat und Polyphenylendiamin verwendet, wobei die Polyphenylendiamin-Schicht durch Aufpolymerisieren von Phenylendiamin direkt auf die Basiselektrode hergestellt wird. Auch nicht weichgemachtes Polyvinylchlorid (PVC) und Nafion kann direkt aus der Lösung aufgebracht werden.

Als Deckschicht 5 wird häufig eine durch Ätzen mikroporös gemachte Polycarbonatmembran verwendet, wobei die Porenweite typischerweise 0,03 µm bei einer Porosität von weniger als 5% beträgt. Diese Deckmembran ist biokompatibel und durch die geringe Porosität diffusionslimitierend, ist jedoch kein Schutz für den Transport von Enzymen durch die Poren. Zur Verbesserung der Diffusionseingenschaften wird diese Schicht häufig noch laminiert und/oder noch weiter beschichtet. Durch Benetzen mit hydrophoben Weichmachern kann eine sogenannte gestützte Flüssigmembran hergestellt werden.

Eine Deckschicht 5 aus einer porösen Polycarbonatmembran hat den Nachteil, daß sie die darunterliegende Enzymschicht 4 nicht ausreichend vor Proteasen schützen kann. Außerdem kann sie ein Auswaschen der Enzyme aus der Enzymschicht 4 nicht verhindern, da Enzyme durch 0,03 µm große Poren diffundieren können.

Deckschichten werden häufig auf der Enzymschicht mechanisch befestigt. Ist die Deckschicht mit der Enzymschicht kombiniert, muß diese Schicht auf der Basiselektrode mechanisch befestigt werden. Derartige mechanische Befestigungen sind teuer, technisch aufwendig und schaffen auch oft insofern Probleme, als es schwierig ist, die Membran luftblasenfrei auf der darunterliegenden Schicht aufzubringen. Dies beschränkt üblicherweise die konstruktiven Freiheiten bei der Sensorgestaltung, da eine bombierte Elektrodenoberfläche nötig ist, um eine Membran unter mechanischer Spannung auf der Elektrode zu befestigen. Die erforderliche Spannung führt oft zu Rissen und Falten. Des weiteren sind Folienmembranen relativ dick, wodurch die hergestellten Sensoren vergleichsweise geringe elektrische Ströme und lange Ansprechzeiten aufweisen.

Zur Ausbildung der Deckmembran ist ferner bekannt, eine Lösung des Polymers auf der Enzymschicht aufzutragen und das Lösungsmittel abzudampfen. Auf diese Weise können z.B. Deckmembranen aus Nafion, PVC, Polyurethan, Silikon, Polyacrylat (p-HEMA) und Celluloseacetat ausgebildet werden, die ohne mechanische Befestigung, also nur durch Adhäsion, auf der darunterliegenden Schicht haften.

JP 04370755 A beschreibt in diesem Zusammenhang einen Glukose-Biosensor, in welchem das Enzym in einer Matrix eingebettet und von dieser überdeckt wird, welche aus einem Copolymer aus (Poly)vinylchlorid und einer geringen Menge eines weiteren Monomers wie beispielsweise Ethylen oder Vinylacetat ohne Zusatz weiterer Substanzen besteht.

Von den bisher verwendeten Polymeren, welche direkt aus ihrer Lösung auf die Enzymschicht bzw. die Elektrode aufgebracht werden können, sind nur einige wenige, wie z.B. Nafion und Celluloseacetat, gegen elektrochemisch aktive Interferenten selektiv. Viele Polymere sind ferner nur in leicht flüchtigen, aggressiven bzw. toxischen Lösungsmitteln löslich, wie z.B. Celluloseacetat in DMSO und Aceton, PVC in Tetrahydrofuran und Cyclohexanon. Dieser Umstand ist sowohl fertigungstechnisch als auch sicherheitstechnisch relevant. Er ist auch für die Elektrode selbst relevant, da Kunststoffteile von diesen Lösungsmitteln angegriffen werden bzw. Enzyme in der Enzymschicht zerstört werden können. Aus weichgemachtem PVC können Weichmacher in umgebende Kunststoffteile bzw. in die Enzymschicht diffundieren.

Ein weiterer Nachteil besteht darin, daß die Permeabilität für die Analyte, also z.B. Glucose oder Laktat, bei den meisten Polymeren, vor allem für weichgemachtes PVC, nur über eine Variation der Schichtdicke eingestellt werden kann, da die Permeabilität hauptsächlich durch Fehlstellen in der Membran und der daduch entstandenen Porosität bedingt ist. Bereits geringe Unterschiede in der Schichtdicke können dabei zu einem totalen Permeabilitätsverlust führen.

Die vorliegende Erfindung stellt sich die Aufgabe, die oben genannten Nachteile bei einem eingangs erwähnten Sensor zu überwinden und insbesondere einen Sensor zur Verfügung zu stellen, bei dem die Deckschicht nicht mechanisch befestigt zu werden braucht. Bei der Aufbringung der Deckschicht aus der Lösung soll ferner keines der aggressiven, toxischen bzw. extrem leichtflüchtigen Lösungsmittel, die im Stand der Technik verwendet werden, eingesetzt werden.

Der erfindungsgemäße Sensor zur Bestimmung der Konzentration und zum Nachweis eines Enzymsubstrates in einer flüssigen Probe, welcher Sensor eine Enzymschicht, deren Enzym mit dem Enzymsubstrat unter Bildung einer direkt oder indirekt detektierbaren Substanz reagieren kann, eine Detektionsvorrichtung und eine für das Enzymsubstrat permeable, auf der Enzymschicht angeordnete Deckmembran aus einem Polymer aufweist, wobei das Polymer ein Polyvinylchlorid-Copolymer, d.h. ein Copolymer aus Vinylchlorid und einem weiteren Monomer, ist, welches Copolymer hydrophile Gruppen aufweist, dadurch gekennzeichnet, dass die Deckmembran das weitere Monomer in einer Menge von 5 bis 35 Masse %, bezogen auf die Masse an Vinylchlorid, und einen hydrophilen Weichmacher in einer Menge von 0,5 bis 5 Masse%, bezogen auf das Polyvinylchlorid-Copolymer, enthält. Das zur Bildung der Deckmembran erfindungsgemäß verwendete Copolymer kann ein Block-Copolymer, ein alternierendes Copolymer oder ein statistisches Copolymer sein.

Als hydrophile Gruppen sind Hydroxy-, Ester- und/oder Carboxylgruppen bevorzugt.

Das erfindungsgemäß verwendete PVC-Copolymer ist in hochsiedenden Estern leicht löslich. Dies ermöglicht ein automatisiertes Dispensieren über mehrere Stunden. Demgegenüber wird reines PVC üblicherweise in Tetrahydrofuran gelöst, das aufgrund seiner hohen Flüchtigkeit kleine reproduzierbare, automatisierte Massenfertigung zuläßt.

Ein weiterer Vorteil ist, daß das gelöste Polymer aus dieser Lösung direkt auf die Enzymschicht aufgebracht werden kann. Eine Inaktivierung der Enzymschicht durch die erfindungsgemäße Deckschicht ist praktisch ausgeschlossen, da gegenüber nicht bedeckten Sensoren nur ca. die Hälfte an Signal verlorengeht. Bemerkenswert ist dabei aber, daß die Linearität des Sensors vervielfacht wird. Eine vergleichbare Deckmembran, z.B. aus Nafion, weist zwar einen ähnlichen Signalverlust auf, jedoch ohne signifikante Erhöhung der Linearität des Sensors.

Das erfindungsgemäß verwendete Copolymer ist insbesondere aus Vinylchlorid und einem oder mehreren von Vinylalkohol, Vinylester, z.B. Vinylacetat, Vinylhydroxyester, z.B. Hydroxypropylacrylat, und Vinylcarbonsäuren, z.B. Maleinsäure, gebildet. Beispielhafte PVC-Copolymere sind Poly-(vinylchlorid-co-vinylacetat), Poly-(vinylchlorid-co-vinylacetat-co-2-hydroxypropylacrylat, Poly-(vinylchlorid-co-vinylacetat-co-maleinsäure und Poly-(vinylchlorid-co-vinylacetat-co-vinylalkohol.

Eine besondere Ausführungsform des erfindungsgemäßen Sensors ist dadurch gekennzeichnet, daß das weitere Monomer, aus welchem das Polyvinylchlorid-Copolymer gebildet ist, Vinylacetat und/oder Maleinsäure ist.

Das weitere Monomer ist im Copolymer vorzugsweise zu 10 bis 20 Masse%, bezogen auf die Masse an Vinylchlorid, enthalten.

Eine weitere bevorzugte Ausführungsform des erfindungsgemäßen Sensors besteht darin, daß als Weichmacher insbesondere Dextran, Glycerin, Polyethylenglycol oder Glycole enthalten sind.

Über den Zusatz an Weichmacher kann die Permeabilität gezielt eingestellt werden. Ein Zusatz von z.B. 3 Masse% Glycerin erhöht bei einem amperometrischen Sensor bei gleicher Schichtdicke das Sensorsignal z.B. auf Laktat um 100% bei einer gleichzeitigen Erhöhung der Linearität um ca. 10%. Auch bei Anwendung bei einem optischen Sensor wird der Meßbereich nach Aufbringen der erfindungsgemäßen Deckschicht signifikant erhöht.

Im Vergleich zu einer Deckmembran aus reinem PVC gestattet die erfindungsgemäße Deckmembran aus dem PVC-Copolymer eine wesentlich bessere Einstellung der Permeabilität für Glucose, da durch die hydrophile Copolymerkomponente a priori eine bessere Permeabilität für Glucose gegeben ist. Es hat sich gezeigt, daß bei Verdoppelung der Schichtdicke die Signalintensität maximal um die Hälfte abnimmt, wogegen bei einer Deckmembran aus reinem PVC eine Verdoppelung der Schichtdicke zu einem totalen Permeabilitätsverlust führen kann.

Die erfindungsgemäße Deckmembran erschwert das Auswaschen von Enzymen oder anderen Bestandteilen der Enzymschicht, wodurch sich die Lebensdauer des Sensors verlängert. Es konnte z.B. die Lebensdauer eines Laktatsensors von 1,5 Tagen (ohne Deckmembran) auf 14 Tage (mit Deckmembran) verlängert werden.

Der erfindungsgemäße Sensor kann als Enzymelektrode ausgebildet sein, wobei die Detektionsvorrichtung eine Basiselektrode ist.

Die Deckmembran kann dabei direkt, das heißt ohne zwischenliegende Interferenzsperrmembran, auf die Basiselektrode bzw. auf eine Schicht, welche das Enzym enthält, aufgebracht werden. Dies deshalb, weil die erfindungsgemäße Deckmembran sowohl die Linearität des Sensors aufgrund einer verbesserten Diffusionshemmung erhöht als auch elektroaktive Interferenten aussperrt. Mit der erfindungsgemäßen Deckmembran konnte der Einfluß von 1 mmol/l Paracetamol von 1,4 mmol/l Laktat ohne Deckmembran auf 0,2 mmol/l Laktat herabgesetzt werden. Desgleichen hat sich die Linearität des Sensors (ein Verhältnis der Meßwerte bei 5 und 12 mmol/l Laktat entspricht absoluter Linearität) von 1,1 auf 2,2 erhöht.

Die Deckmembran kann durch Adhäsion an der Basiselektrode bzw. an der Schicht, welche das Enzym enthält, haften und braucht nicht als Folie auf mechanische Weise an der Basiselektrode bzw. an der Schicht, welche das Enzym enthält, angebracht sein.

Der erfindungsgemäße Sensor kann ferner als optischer Sensor ausgebildet sein.

Die Erfindung betrifft auch eine Methode zur Bestimmung der Konzentration und zum Nachweis eines Enzymsubstrates in einer flüssigen Probe mittels eines Sensors, der mit der flüssigen Probe in Kontakt gebracht wird, welche Methode dadurch gekennzeichnet ist, daß ein erfindungsgemäßer Sensor eingesetzt wird.

Die Erfindung betrifft ferner die Verwendung eines Polyvinylchlorid-Copolymers, welches aus Vinylchlorid und einem weiteren Monomer in einer Menge von 5 bis 35 Masse%, bezogen auf die Masse an Vinylchlorid, besteht und hydrophile Gruppen aufweist, sowie einen hydrophilen Weichmacher in einer Menge von 0,5 bis 5 Masse %, bezogen auf das Polyvinylchlorid-Copolymer, enthält, zur Herstellung einer Deckmembran eines Sensors.

Mit der beigefügten Zeichnung werden bevorzugte Ausführungsformen der Erfindung noch näher beschrieben, wobei die Figuren 2 und 3 schematisch einen Querschnitt durch eine erfindungsgemäße Enzymelektrode bzw. einen erfindungsgemäßen optischen Sensor (Optode) zeigen.

Die Figur 2 zeigt den Aufbau einer bevorzugten Ausführungsform einer erfindungsgemäßen Enzymelektrode, wobei die Bezugsziffer 6 eine elektrisch leitende Bahn, z.B. aus Silber, die auf einem Kunststoffträger (nicht dargestellt) aufgebracht ist, bezeichnet. Die Basiselektrode 7 ist auf die Bahn 6 aufgebracht. Über der Basiselektrode 7 ist die Enzymschicht 8 und die erfindungsgemäße Deckmembran 9, die mit der Probe in Kontakt steht, vorgesehen. Der Schichtaufbau für eine derartige Enzymelektrode kann folgendermaßen vorgenommen werden.

Zunächst werden auf den Kunststoffträger Silberleitbahnen für die Referenzelektrode, die Gegenelektrode und die erfindungsgemäße Enzymelektrode (Basiselektrode bzw. Arbeitselektrode) gedruckt.

Die Referenzelektrode wird weiters im Sensorbereich aus einer Ag/AgCl-Paste hergestellt. Die Gegenelektroden werden mit einer Carbonpaste im Sensorbereich überschichtet. Als Basis für die Enzymelektrode wird eine Mischung aus 5% MnO₂ in einer Carbonpaste im Sensorbereich aufgedruckt. Darauf wird ein Tropfen einer 10%igen Lösung von Glucoseoxidase zur Bestimmung von Glucose in Wasser aufgebracht, oder alternativ in einer selbstvernetzenden Polyacrylmatrix.

Anschließend wird die Deckmembran aufgetragen, indem eine 1-3%ige Lösung des erfindungsgemäß verwendeten Polyvinylchlorid-Copolymers in Butoxyethylacetat, z.B. PVC-co-vinylacetat-maleinat, ein- bis dreimal hintereinander aufgetropft und das Lösungsmittel abgedampft wird.

Zur Permeabilitätsverbesserung und zur Verhinderung einer möglichen Kristallisationsbildung in der Polyvinylchlorid-Copolymer-Membran werden hydrophile Weichmacher (Glycerin, PEG, Dextran oder ähnliches), zweckmäßigerweise in einer Konzentration von 3% des Polyvinylchlorid-Copolymers, zugesetzt.

Statt der Glucoseoxidase können auch andere Enzyme zur Bestimmung anderer Substrate, z.B. Laktatoxidase zur Bestimmung von Laktat, eingesetzt werden.

Die Figur 3 zeigt schematisch den Aufbau eines optischen Sensors, wobei die Bezugsziffer 10 einen Träger, z.B. aus Polyester, bezeichnet, die Bezugsziffer 11 eine Silikonschicht bezeichnet, die einen Indikator, z.B. Decacyclen zur Detektion von Sauerstoff, enthält, die Bezugsziffer 12 eine Schicht aus feinverteiltem Kohlenstoff zur optischen Isolierung bezeichnet und die Bezugsziffer 13 eine Enzymschicht (Gelschicht) bezeichnet. Die Herstellung eines derartigen Sensors ist z.B. aus Biosensors & Bioelectronics 5 (1990) 137-148 bekannt.

Zur Aufbringung der Deckschicht 14 aus dem Polyvinylchlorid-Copolymer wird wie oben bereits beschrieben vorgegangen. Diese Deckschicht 14 steht mit der Probe in Kontakt.

Der zu bestimmende Stoff, z.B. Glucose, gelangt durch die Deckmembran 14 in die Enzymschicht 13, welche Glucoseoxidase enthält. Der durch enzymatische Oxidation von Glucose entstehende Sauerstoff gelangt in die Indikatorschicht 11 und löscht die Fluoreszenz des Decacyclens, die mittels einer Anregungseinrichtung 15, welche Anregungslicht in die Silikonschicht 11 strahlt, hervorgerufen wird. Aus der Abnahme der Fluoreszenz kann in an sich bekannter Weise mittels der Meßeinrichtung 16, der ein Filter 17 vorgeschaltet ist, auf die Sauerstoffkonzentration und in der Folge auf die Glucosekonzentration rückgeschlossen werden.

## Patentansprüche

1. Sensor zur Bestimmung der Konzentration und zum Nachweis eines Enzymsubstrates in einer flüssigen Probe, welcher Sensor eine Enzymschicht (8), deren Enzym, mit dem Enzymsubstrat unter Bildung einer direkt oder indirekt detektierbaren Substanz reagieren kann, eine Detektionsvorrichtung (7) und eine für das Enzymsubstrat permeable, auf der Enzymschicht (8) angeordnete Deckmembran (9) aus einem Polymer aufweist, wobei das Polymer ein Polyvinylchlorid-Copolymer, d.h. ein Copolymer aus Vinylchlorid und einem weiteren Monomer, ist, welches Copolymer hydrophile Gruppen aufweist, **dadurch gekennzeichnet, dass** die Deckmembran das weitere Monomer zu 5 bis 35 Masse %, bezogen auf die Masse an Vinylchlorid, und einen hydrophilen Weichmacher in einer Menge von 0,5 bis 5 Masse%, bezogen auf das Polyvinylchlorid-Copolymer, enthält.

2. Sensor nach Anspruch 1, **dadurch gekennzeichnet, daß** das Copolymer als hydrophile Gruppen Hydroxy-, Ester- und/oder Carboxylgruppen aufweist.

3. Sensor nach Anspruch 2, **dadurch gekennzeichnet, daß** das Copolymer aus Vinylchlorid und einem oder mehreren von Vinylalkohol, Vinylester, Vinylhydroxyester und Vinylcarbonsäuren gebildet ist.

4. Sensor nach Anspruch 3, **dadurch gekennzeichnet, daß** das weitere Monomer Vinylacetat und/oder Maleinsäure ist.

5. Sensor nach Anspruch 1 bis 4, **dadurch gekennzeichnet, daß** als Weichmacher Dextran, Glycerin, Polyethylenglycol oder Glycole enthalten sind.

6. Sensor nach einem der Ansprüche 1 oder 5, **dadurch gekennzeichnet, daß** dass das weitere Monomer zu 10 bis 20 Masse%, bezogen auf die Masse an Vinylchlorid, enthalten ist.

7. Sensor nach einem der Ansprüch 1 bis 6, **dadurch gekennzeichnet, dass** er als Enzymelektrode ausgebildet ist, wobei die Detektionsvorrichtung eine Basiselektrode ist.

8. Sensor nach Anspruch 7, **dadurch gekennzeichnet, dass** die Deckmembran direkt, das heißt ohne zwischenliegende Interferenzsperrmembran, auf die Basiselektrode bzw. auf eine Schicht, welche das Enzym enthält, aufgebracht ist.

9. Sensor nach Ansprüch 8, **dadurch gekennzeichnet, dass** die Deckmembran durch Adhäsion an der Basiselektrode bzw. auf der Schicht, welche das Enzym enthält, haftet.

10. Sensor nach einem der Ansprüche 1 bis 6, **dadurch gekennzeichnet, dass** er als optischer Sensor ausgebildet ist.

11. Methode zur Bestimmung der Konzentration und zum Nachweis eines Enzymsubstrates in einer flüssigen Probe mittels eines Sensors, der mit der flüssigen Probe in Kontakt gebracht wird, **dadurch gekennzeichnet, dass** ein Sensor gemäß einem der Ansprüche 1 bis 10 eingesetzt wird.

12. Verwendung eines Polyvinylchlorid-Copolymers, welches aus Vinylchlorid und einem weiteren Monomer in einer Menge von 5 bis 35 Masse%, bezogen auf die Masse an Vinylchlorid, besteht und hydrophile Gruppen aufweist, sowie einen hydrophilen Weichmacher in einer Menge von 0,5 bis 5 Masse %, bezogen auf das Polyvinylchlorid-Copolymer, enthält, zur Herstellung einer Deckmembran eines Sensors

## Claims

1. A sensor for the determination of the concentration and the detection of an enzyme substrate in a liquid sample, said sensor comprising an enzyme layer (8) whose enzyme is capable of reacting with said enzyme substrate while producing a substance directly or indirectly detectable, a detection device (7) and a cover membrane (9) from a polymer, which cover membrane is arranged on the enzyme layer (8) and is permeable to said enzyme substrate, wherein the polymer is a polyvinyl chloride copolymer, i.e. a copolymer from vinyl chloride and a further monomer, said copolymer comprising hydrophilic groups, **characterized in that** the cover membrane contains the further monomer at 5 to 35% by mass, based on the mass of vinyl chloride, and a hydrophilic plasticizer in an amount of from 0.5 to 5% by mass, based on the polyvinyl chloride copolymer.

2. The sensor according to claim 1, **characterized in that** the copolymer comprises hydroxy, ester and/or carboxyl groups as said hydrophilic groups.

3. The sensor according to claim 2, **characterized in that** the copolymer is made from vinyl chloride and one or more of vinyl alcohol, vinyl ester, vinyl hydroxy ester and vinyl carboxylic acids.

4. The sensor according to claim 3, **characterized in that** the further monomer is vinyl acetate and/or maleic acid.

5. The sensor according to any of claims 1 to 4, **characterized in that** dextrane, glycerin, polyethylene glycol or glycols are contained as said plasticizer.

6. The sensor according to any of claims 1 to 5, **characterized in that** the further monomer is contained in an amount of from 10 to 20% by mass, based on the mass of vinyl chloride.

7. The sensor according to any of claims 1 to 6, **characterized in that** it is designed as an enzyme electrode, said detection device being a base electrode.

8. The sensor according to claim 7, **characterized in that** the cover membrane is applied directly, i.e. without intermediate interference-blocking membrane, onto said base electrode or onto a layer containing the enzyme, respectively.

9. The sensor according to claim 8, **characterized in that** the cover membrane sticks by adhesion to the base electrode or to the layer containing the enzyme, respectively.

10. The sensor according to any of claims 1 to 6, **characterized in that** it is designed as an optical sensor.

11. A method for the determination of the concentration and the detection of an enzyme substrate in a liquid sample by means of a sensor contacted with said liquid sample, **characterized in that** a sensor according to any of claims 1 to 10 is employed.

12. The use of a polyvinyl chloride copolymer consisting of vinyl chloride and a further monomer in an amount of from 5 to 35% by mass, based on the mass of vinyl chloride, and comprising hydrophilic groups as well as containing a hydrophilic plasticizer in an amount of from 0.5 to 5% by mass, based on the polyvinyl chloride copolymer, for the preparation of a cover membrane of a sensor.

## Revendications

1. Capteur pour déterminer la concentration et pour mettre en évidence un substrat d'enzyme dans un échantillon liquide, lequel capteur comporte une couche d'enzyme (8) dont l'enzyme peut réagir avec le substrat d'enzyme en formant une substance détectable directement ou indirectement, un dispositif de détection (7) et une membrane de couverture (9) en un polymère, perméable au substrat d'enzyme, disposée sur la couche d'enzyme (8), où le polymère est un copolymère de poly(chlorure de vinyle), c'est à dire un copolymère de chlorure de vinyle et d'un autre monomère, lequel copolymère comporte des groupes hydrophiles, **caractérisé en ce que** la membrane de couverture contient l'autre monomère à raison de 5 à 35 % en masse, par rapport à la masse de chlorure de vinyle, et un plastifiant hydrophile en une quantité de 0,5 à 5 % en masse, par rapport au copolymère de poly(chlorure de vinyle).

2. Capteur selon la revendication 1 **caractérisé en ce que** le copolymère comporte comme groupes hydrophiles des groupes hydroxy, ester et/ou carboxyle.

3. Capteur selon la revendication 2 **caractérisé en ce que** le copolymère est formé de chlorure de vinyle et d'un ou plusieurs parmi l'alcool vinylique, un ester de vinyle, un hydroxyester de vinyle et des acides vinylcarboxyliques.

4. Capteur selon la revendication 3 **caractérisé en ce que** l'autre monomère est l'acétate de vinyle et/ou l'acide maléique.

5. Capteur selon l'une des revendications 1 à 4 **caractérisé en ce que** le dextrane, la glycérine, le polyéthylèneglycol ou des glycols sont contenus comme plastifiant.

6. Capteur selon l'une des revendications 1 à 5 **caractérisé en ce que** l'autre monomère est contenu à raison de 10 à 20 % en masse, par rapport à la masse de chlorure de vinyle.

7. Capteur selon l'une des revendications 1 à 6 **caractérisé en ce qu'**il est agencé sous forme d'électrode enzymatique, où le dispositif de détection est une électrode de base.

8. Capteur selon la revendication 7 **caractérisé en ce que** la membrane de couverture est appliquée directement, c'est à dire sans membrane d'arrêt à interférence intermédiaire, sur l'électrode de base ou sur une couche qui contient l'enzyme.

9. Capteur selon la revendication 8 **caractérisé en ce que** la membrane de couverture est fixée par adhésion à l'électrode de base ou à la couche qui contient l'enzyme.

10. Capteur selon l'une des revendications 1 à 6 **caractérisé en ce qu'**il est agencé sous forme de capteur optique.

11. Méthode de détermination de la concentration et de mise en évidence d'un substrat d'enzyme dans un échantillon liquide au moyen d'un capteur qui est mis en contact avec l'échantillon liquide, **caractérisée en ce qu'**un capteur selon l'une des revendications 1 à 10 est utilisé.

12. Utilisation d'un copolymère de poly(chlorure de vinyle) qui consiste en chlorure de vinyle et en un autre monomère en une quantité de 5 à 35 % en masse, par rapport à la masse de chlorure de vinyle, et comporte des groupes hydrophiles, et contient un plastifiant hydrophile en une quantité de 0,5 à 5 % en masse, par rapport au copolymère de poly(chlorure de vinyle), pour produire une membrane de couverture d'un capteur.
